# EUROPÄISCHE PATENTSCHRIFT

(11) **EP 2 906 574 B1**
(45) Veröffentlichungstag und Bekanntmachung des Hinweises auf die Patenterteilung: **02.11.2016**
(21) Anmeldenummer: 13773210.3
(22) Anmeldetag: 27.09.2013
(51) Int. Cl.: C07F 9/6574, B01J 31/02, B01J 31/18, C07C 45/50

(54) **GEMISCH AUS VERSCHIEDENEN UNSYMMETRISCHEN BISPHOSPHITEN UND DESSEN VERWENDUNG ALS KATALYSATORGEMISCH IN DER HYDROFORMYLIERUNG**
MIXTURE OF DIFFERENT ASYMMETRICAL BIPHOSPHITES AND ITS USE AS CATALYST MIXTURE IN THE HYDROFORMYALTION
MÉLANGE DE BIPHOSPHITES ASYMÉTRIQUES DIFFÉRENTS ET SON UTILISATION COMME MÉLANGE CATALYTIQUE POUR L'HYDROFORMYLATION

(30) Priorität: 12.10.2012 DE 102012218627; 12.10.2012 DE 102012218625; 12.10.2012 DE 102012218629; 12.10.2012 DE 102012218630
(43) Veröffentlichungstag der Anmeldung: 19.08.2015
(73) Patentinhaber: Evonik Degussa GmbH, 45128 Essen (DE)
(72) Erfinder: CHRISTIANSEN, Andrea, 18119 Rostock (DE); FRANKE, Robert, 45772 Marl (DE); FRIDAG, Dirk, 45721 Haltern am See (DE); HESS, Dieter, 45770 Marl (DE); DYBALLA, Katrin Marie, 45657 Recklinghausen (DE); HANNEBAUER, Bernd, 63165 Mühlheim (DE)
(86) Internationale Anmeldenummer: PCT/EP2013/070238
(87) Internationale Veröffentlichungsnummer: WO 2014/056737

(56) Entgegenhaltungen:
- EP-B1- 1 294 731
- US-A- 4 769 498

## Beschreibung

Die Erfindung betrifft ein Gemisch aus unsymmetrischen Bisphosphiten, ein Verfahren zu dessen Herstellung, sowie deren Umsetzung mit Metallen zu Gemischen enthaltenden Komplexverbindungen aus den Bisphosphiten und dem Metall, sowie deren Verwendung als katalytisch aktive Zusammensetzung in Hydroformylierungsreaktionen, wie auch die Hydroformylierungsreaktion selbst.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Katalytisch aktive Zusammensetzungen auf Basis von Rhodium-Bisphosphit-Komplexen sind geeignet für die Hydroformylierung von linearen Olefinen mit end- und innenständigen Doppelbindungen, wobei überwiegend endständig hydroformylierte Produkte entstehen. Dagegen werden verzweigte Olefine mit innenständigen Doppelbindungen nur im geringen Masse umgesetzt. Diese Phosphite ergeben bei ihrer Koordination an ein Übergangsmetallzentrum Katalysatoren von gesteigerter Aktivität, doch ist das Standzeitverhalten dieser katalytisch aktiven Zusammensetzungen, unter anderem wegen der Hydrolyseempfindlichkeit der Phosphitliganden, unbefriedigend. Durch den Einsatz von substituierten Bisaryldiolen als Bausteine für die Phosphitliganden, wie in EP 0 214 622 oder EP 0 472 071 beschrieben, konnten erhebliche Verbesserungen erreicht werden.

Der Literatur zufolge sind die katalytisch aktiven Zusammensetzungen dieser Liganden auf Basis von Rhodium äußerst aktiv in der Hydroformylierung von α-Olefinen. In den Patenten US 4 668 651, US 4 748 261 und US 4 885 401 werden Polyphosphitliganden beschrieben, mit denen α-Olefine, aber auch 2-Buten mit hoher n/i-Selektivität zu den terminal oxierten Produkten umgesetzt werden können. Zweizähnige Liganden dieses Typs wurden auch zur Hydroformylierung von Butadien eingesetzt (US 5 312 996).

Die in EP 1 294 731 offenbarten Bisphosphite weisen bei der Hydroformylierung von n-Octengemischen Olefinumsätze bis zu 98 % auf. Jedoch ist die ebenfalls gewünschte n-Selektivität zum Nonanal mit 36,8 % bis maximal 57,6 % verbesserungswürdig. Dies gilt umso mehr, als dass die Verwendung der katalytisch aktiven Zusammensetzung in technischen Prozessen eine Standzeit verlangt, welche sich in Tagen anstelle von Stunden bemisst. Literaturbekannt ist die Synthese symmetrisch aufgebauter Bisphosphite, wie sie seit US 4 769 498 offenbart wurden und deren Verwendung in katalytisch aktiven, übergangsmetallhaltigen Zusammensetzungen zur Hydroformylierung ungesättigter Verbindungen.

In US 4 769 498, wie auch in US 5 723 641 werden bevorzugt symmetrisch aufgebaute Bisphosphite hergestellt und als Liganden zur Hydroformylierung verwendet. Die in der Hydroformylierung verwendeten symmetrisch aufgebauten Bisphosphitliganden werden bei tiefen Temperaturen hergestellt. Die Einhaltung dieser tiefen Temperaturen ist zwingend erforderlich, da höhere Temperaturen gemäß dieser US-Schriften zu Umlagerungen und letztlich zu unsymmetrisch aufgebauten Bisphosphiten führen würde, was aber hier nicht erwünscht ist.

Bei den in US 5 288 918 in Spalte 8 unter der allgemeinen Formel (V) offenbarten Bisphosphiten handelt es sich um symmetrische Bisphosphite. Das Bisphosphit ist selbst dann symmetrisch, wenn X¹ und X² für unterschiedliche Reste stehen, wie es in der Tabelle in Spalte 11 bei Ref.No. 2 und 3 der Fall ist.
Normalerweise werden im Stand der Technik möglichst reine Liganden in der Hydroformylierungsreaktion eingesetzt, da das jeweils andere Isomer starke negative Einflüsse auf die Gesamtperformance des Systems ausübt. In der Regel würde das unsymmetrische Isomer als Nebenkomponente vorliegen, da ausschließlich symmetrische Liganden in der Hydroformylierung eingesetzt werden, In WO 2007/149143 wird ein Gemisch aus zwei Monophosphiten beschrieben, welches als Antioxidans für Polymerharze verwendet wird.

Der Einsatz von zwei unsymmetrischen Bisphosphiten in der Hydroformylierung ist derzeit noch nicht beschrieben.

Die technische Aufgabe der vorliegenden Erfindung ist die Bereitstellung neuer Liganden, welche in der Hydroformylierung von ungesättigten Verbindungen nicht die aus dem Stand der Technik zuvor aufgezeigten Nachteile, sondern die folgenden Eigenschaften aufweisen:
1.) eine hohe Aktivität, und
2.) eine hohe n-Regioselektivität in Bezug auf die Hydroformylierung und
3.) eine hohe Standzeit.

Eine hohe Standzeit bedeutet, dass die hydroformylierungsaktive Zusammensetzung, welche die Liganden neben weiteren Komponenten umfasst, eine geringe Tendenz zum Abbau dieser Liganden und/oder dem Zerfall dieser Liganden in hydroformylierungs-inhibierende Komponenten, wie z.B. die sogenannten *"Poisoning Phosphites"* aufweist.

Die Aufgabe wird gelöst durch ein Gemisch von unsymmetrischen Bisphosphiten umfassend die Verbindungen (Ia') und (Ia"): 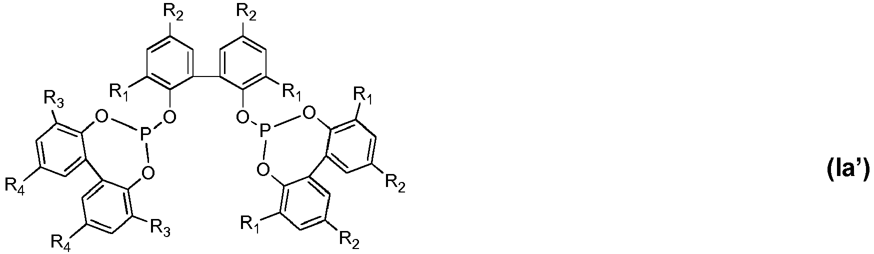 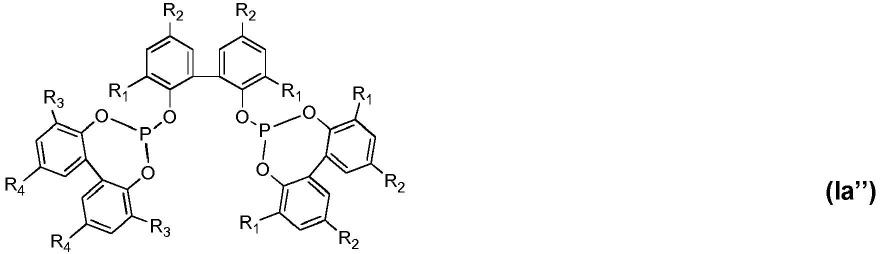 wobei
R1 ausgewählt ist aus -Me, -tBu, -OMe;
R2 ausgewählt ist aus -Me, -tBu, -OMe;
R3 ausgewählt ist aus -Me, -tBu, -OMe;
R4 ausgewählt ist aus -Me, -tBu, -OMe;
und P weitere Bindungen eingehen kann,
und die Verbindungen (Ia') und (Ia") nicht identisch sind.

Sowohl Ia' wie auch Ia" ist jeweils unsymmetrisch. Es liegt also ein Gemisch von zwei unterschiedlichen, unsymmetrischen Bisphosphiten vor. Unsymmetrisch bedeutet, dass für den Fall, dass R1 gleich R3 ist, nicht gleichzeitig R2 gleich R4 sein kann. Oder für den Fall, dass R2 gleich R4 ist, R1 nicht gleichzeitig R3 sein kann.

In einer Ausführungsform liegt der Gehalt an Verbindung (Ia') in einem Bereich von 99,5 bis 0,5 Mol-%, der Gehalt an Verbindung (Ia") in einem Bereich von 0,5 bis 99,5 Mol-%.
Die beiden Verbindungen (Ia') und (Ia") addieren sich zu 100 Mol-%.

Exemplarisch für die verschiedenen Reste R sind die Verbindungen (1Ia') und (1Ia") in der nachfolgenden Tabelle 1 aufgeführt.

**Tabelle 1:**

| | Isomer | R1 | R2 | R3 | R4 |
|---|---|---|---|---|---|
| (1Ia') | (Ia') | -Me | -Me | -tBu | -OMe |
| (1Ia") | (Ia") | -tBu | -OMe | -Me | -Me |

In einer Ausführungsform umfasst das Gemisch die Verbindungen Ib' und Ib": 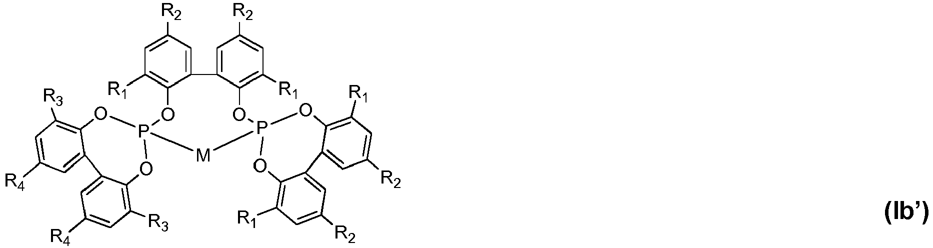 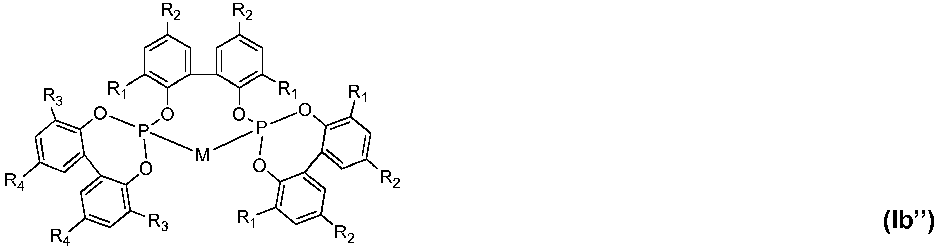 wobei M ausgewählt ist aus Fe, Ru, Os, Co, Rh, Ir, Ni, Pd, Pt,
und M zusätzliche Bindungen eingehen kann,
und die Verbindungen (Ib') und (Ib") nicht identisch sind.

In einer Ausführungsform liegt der Gehalt an Verbindung (Ib') in einem Bereich von 99,5 bis 0,5 Mol-%, der Gehalt an Verbindung (Ib") in einem Bereich von 0,5 bis 99,5 Mol-%.
Die beiden Verbindungen (Ib') und (Ib") addieren sich zu 100 Mol-%.

In einer Ausführungsform umfasst das Gemisch die Verbindungen Ic' und Ic": 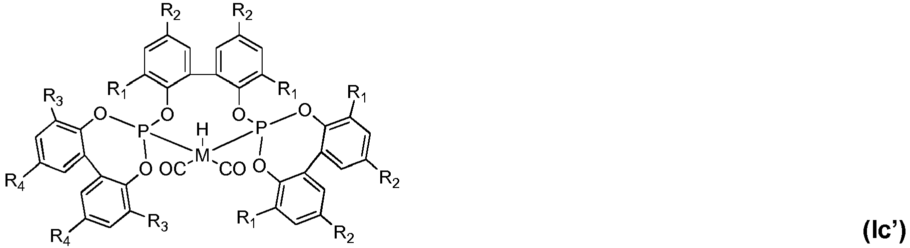 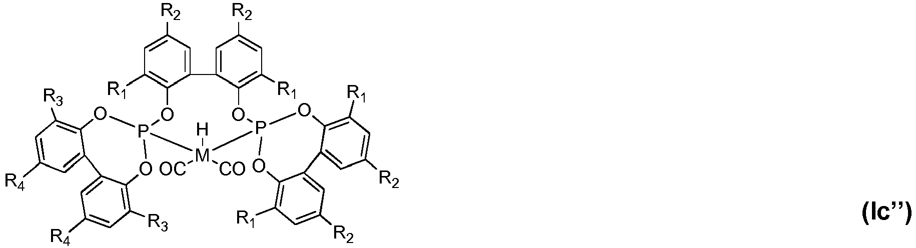 wobei M ausgewählt ist aus Fe, Ru, Os, Co, Rh, Ir, Ni, Pd, Pt, und
und die Verbindungen (Ic') und (Ic") nicht identisch sind.

In einer Ausführungsform liegt der Gehalt an Verbindung (Ic') in einem Bereich von 99,5 bis 0,5 Mol-%, der Gehalt an Verbindung (Ic") in einem Bereich von 0,5 bis 99,5 Mol-%.
Die beiden Verbindungen (Ic') und (Ic") addieren sich zu 100 Mol-%.

In einer Ausführungsform umfasst das Gemisch mindestens eine Verbindung (Ia') oder (Ia"), die nicht an M gebunden sind.

In einer Ausführungsform steht M für Rh.

In einer Ausführungsform ist R1 -Me, und R3 nicht -Me.

In einer Ausführungsform ist R2 -Me, und R4 nicht -Me.

In einer Ausführungsform sind R1 und R2 -Me.

In einer Ausführungsform ist R1 -tBu ist, und R3 nicht -tBu.

In einer Ausführungsform ist R2 -OMe, und R4 nicht -OMe.

In einer bevorzugten Ausführungsform weisen die Bisphosphite die Strukturen (1Ia') und (1Ia") auf:

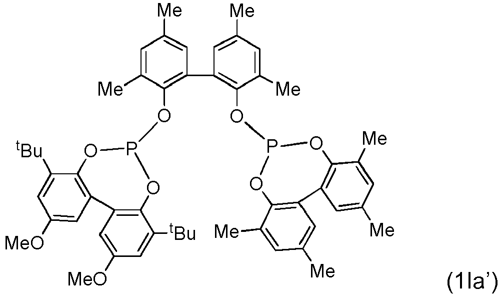

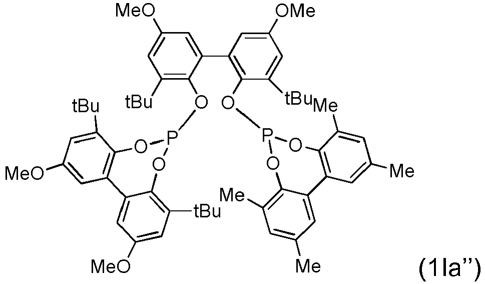

Neben dem Gemisch wird auch eine Zusammensetzung beansprucht, welche dieses umfasst.

Zusammensetzung umfassend:
- ein zuvor beschriebenes Gemisch,
- eine weitere Komponente ausgewählt aus: Basen, organische Amine, Epoxide, Pufferlösungen, Ionenaustauscher.

In einer bevorzugten Ausführungsform werden als weitere Komponenten sterisch gehinderte sekundäre Amine eingesetzt.

Es können auch Gemische, enthaltend zwei oder mehrere sterisch gehinderte Amine, eingesetzt werden.

Die Zusammensetzung umfasst ein zuvor beschriebenes Gemisch, welche zusätzlich zu dem Gemisch zumindest ein Amin mit einer 2,2,6,6-Tetramethylpiperidineinheit aufweist.

Insbesondere wird im erfindungsgemäßen Verfahren das Amin mit der Formel (7), Sebacinsäuredi-4-(2,2,6,6-tetramethylpiperidinyl)ester, bevorzugt eingesetzt.

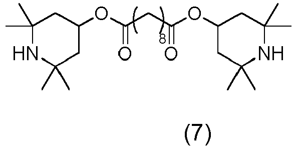

Ein besonders bevorzugtes Metall in der erfindungsgemäßen Zusammensetzung ist Rhodium.

Neben dem Gemisch selbst, wird auch dessen Verwendung als Katalysator in einer Hydroformylierungsreaktion von ungesättigten Verbindungen und deren Gemischen beansprucht.

Des Weiteren wird ein Verfahren zur Hydroformylierung von ungesättigten Verbindungen und deren Gemischen beansprucht.

Verfahren zur Hydroformylierung von ungesättigten Verbindungen und deren Gemischen unter Verwendung:
- einer zuvor beschriebene Zusammensetzung, und
- eines Gasgemisches umfassend Kohlenmonoxid und Wasserstoff.

In einer Variante des Verfahrens sind die ungesättigten Verbindungen und deren Gemische ausgewählt aus:
- Kohlenwasserstoffgemischen aus Dampfspaltanlagen;
- Kohlenwasserstoffgemischen aus katalytisch betriebenen Spaltanlagen;
- Kohlenwasserstoffgemischen aus Oligomerisierungsprozessen;
- Kohlenwasserstoffgemischen umfassend mehrfach ungesättigte Verbindungen;
- ungesättigten Carbonsäurederivaten.

Die ungesättigten Verbindungen, welche in dem erfindungsgemäßen Verfahren hydroformyliert werden, umfassen Kohlenwasserstoffgemische, die in petrochemischen Verarbeitungsanlagen anfallen. Hierzu gehören beispielsweise sogenannte C₄-Schnittte. Typische Zusammensetzungen von C₄-Schnitten, aus denen der größte Teil der mehrfach ungesättigten Kohlenwasserstoffe entfernt worden ist und die im erfindungsgemäßen Verfahren eingesetzt werden können, sind in der folgenden Tabelle 1 aufgelistet (siehe DE 10 2008 002188).

**Tabelle 2:**

| | Dampfspaltanlage | | Dampfspaltanlage | | Katalytische Spaltanlage | |
|---|---|---|---|---|---|---|
| Komponente | HCC₄ | HCC₄ / SHP | Raff. I | Raff. I / SHP | CC₄ | CC₄/SHP |
| Isobutan [Massen-%] | 1 - 4.5 | 1 - 4.5 | 1.5 - 8 | 1.5 - 8 | 37 | 37 |
| n-Butan [Massen-%] | 5 - 8 | 5 - 8 | 6 - 15 | 6 - 15 | 13 | 13 |
| E-2-Buten [Massen-%] | 18 - 21 | 18 - 21 | 7 - 10 | 7 - 10 | 12 | 12 |
| 1-Buten [Massen-%] | 35 - 45 | 35 - 45 | 15 - 35 | 15 - 35 | 12 | 12 |
| Isobuten [Massen-%] | 22 - 28 | 22 - 28 | 33 - 50 | 33 - 50 | 15 | 15 |
| Z-2-Buten [Massen-%] | 5 - 9 | 5 - 9 | 4 - 8 | 4 - 8 | 11 | 11 |
| 1,3-Butadien [Massen-ppm] | 500 - 8000 | 0 - 50 | 50 - 8000 | 0 - 50 | < 10000 | 0-50 |

### Erläuterung:

- HCC₄: typisch für eine C₄ Mischung, die aus dem C₄-Schnitt einer Dampfspaltanlage (High Severity) nach der Hydrierung des 1,3-Butadiens ohne zusätzliche Moderation des Katalysators erhalten wird.
- HCC₄ / SHP: Zusammensetzung HCC₄, bei dem Reste an 1,3-Butadien in einem Selektivhydrierungsprozess/SHP weiter reduziert wurden.
- Raff. I (Raffinat I): typisch für eine C₄ Mischung, die aus dem C₄-Schnitt einer Dampfspaltanlage (High Severity) nach der Abtrennung des 1,3-Butadiens, beispielsweise durch eine NMP-Extraktivrektifikation, erhalten wird.
- Raff. I / SHP: Zusammensetzung Raff. I, bei dem Reste an 1,3-Butadien in einem Selektivhydrierungsprozess/SHP weiter reduziert wurden.
- CC₄: typische Zusammensetzung eines C₄-Schnitts, das aus einer katalytischen Spaltanlage erhalten wird.
- CC₄ / SHP: Zusammensetzung eines C₄-Schnitts, bei dem Reste an 1,3-Butadien in einem Selektivhydrierungsprozess/SHP weiter reduziert wurden.

In einer Variante des Verfahrens ist die ungesättigte Verbindung oder deren Gemisch ausgewählt aus:
- Kohlenwasserstoffgemischen aus Dampfspaltanlagen;
- Kohlenwasserstoffgemischen aus katalytisch betriebenen Spaltanlagen, wie z.B. FCC-Spaltanlagen;
- Kohlenwasserstoffgemischen aus Oligomerisierungsprozessen in homogener Phase sowie heterogenen Phasen, wie z.B. dem OCTOL-, DIMERSOL.-, Fischer-Tropsch-, Polygas-, CatPoly-, InAlk-, Polynaphtha-, Selectopol-, MOGD-, COD-, EMOGAS-, NExOCTANE- oder SHOP-Prozess;
- Kohlenwasserstoffgemischen umfassend mehrfach ungesättigte Verbindungen;
- ungesättigten Carbonsäurederivaten.

In einer Variante des Verfahrens weist das Gemisch ungesättigte Verbindungen mit 2 bis 30 Kohlenstoffatomen auf.

In einer besonderen Variante des Verfahrens weist das Gemisch ungesättigte Verbindungen mit 2 bis 8 Kohlenstoffatomen auf.

In einer weiteren Variante des Verfahrens weist das Gemisch mehrfach ungesättigte Kohlenwasserstoffe auf. In einer besonderen Ausführungsform umfasst das Gemisch Butadiene.

Die ungesättigten Verbindungen, welche in dem erfindungsgemäßen Verfahren hydroformyliert werden, umfassen weiterhin ungesättigte Carbonsäurederivate. In einer besonderen Ausführungsform sind diese ungesättigten Carbonsäurederivate ausgewählt unter Fettsäureestern.

Die Durchführung des erfindungsgemäßen Verfahrens erfolgt in unterschiedlichen Ausführungsformen, welche in den Beispielen im Detail offenbart werden.

Das erfindungsgemäße mehrphasige Reaktionsgemisch umfasst neben einem aus Kohlenmonoxid und Wasserstoff bestehenden Gasgemisch mindestens eine ungesättigte Verbindung, wie sie zuvor offenbart wurde und umfasst neben Kohlenwasserstoffgemischen, welche aus Dampfspalt-, katalytisch betriebenen Spaltanlagen oder Oligomerisierungsprozessen stammen oder andere Quellen von einfach ungesättigten und/oder mehrfach ungesättigten Kohlenstoffverbindungen oder ungesättigte Carbonsäurederivate beinhalten, mindestens ein Hydroformylierungsprodukt dieser ungesättigten Verbindungen, wie sie in den nachfolgenden Beispielen aufgeführt sind und die jeweils verwendete Zusammensetzung, wie sie zuvor offenbart wurde.

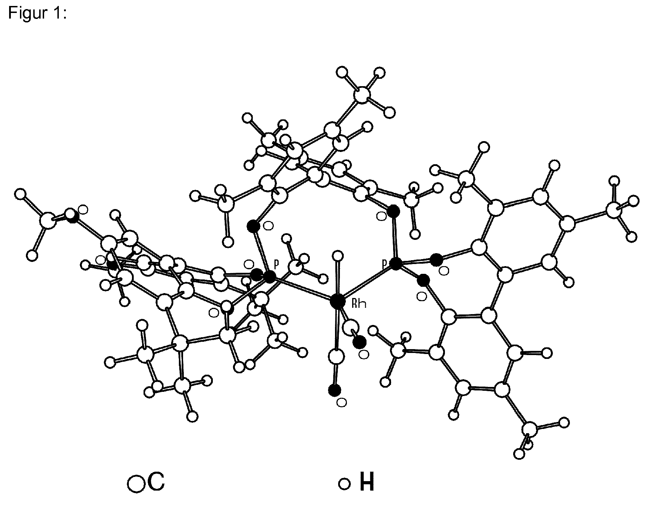

Die Figur 1 zeigt die berechnete Komplexverbindung (Ic') mit R1=Me, R2=Me, R3=tBu, R4=OMe und M=Rh.

Die erfindungsgemäßen Komplexverbindung der Formeln (Ic') und (Ic") werden in-situ während der Hydroformylierungsreaktion gebildet.
In einer besonderen Ausführungsform der Erfindung liegen die Komplexverbindungen (Ic') und (Ic") neben dem ungebundenen Bisphosphit vor.
Die Charakterisierung des Hydridocarbonylkomplexes (Ic') mit Rhodium als Metall erfolgte mittels theoretischer Berechnungen. Das Ergebnis ist in der Figur 1 im Anhang dargestellt.

Die Strukturberechnung wurde mit dem BP86-Funktional und dem def-SV(P)-Basissatz durchgeführt.

Die Strukturberechnungen für die Modellstrukturen erfolgten mit dem Turbomole-Programmpaket (R. Ahlrichs, M. Bär, M. Häser, H. Horn, C. Kölmel, Chem. Phys. Lett., 1989, 162, 16; TURBOMOLE V6.3 2011, a development of University of Karlsruhe and Forschungszentrum Karlsruhe GmbH, 1989-2007, TURBOMOLE GmbH, since 2007. http://www.turbomole.com) auf Basis der Dichtefunktionaltheorie (DFT). Verwendet wurde das BP86-Funktional (S. H. Vosko, L. Wilk, M. Nusair, Can. J. Phys., 1980, 58, 1200; A. D. Becke, Phys. Rev. A, 1988, 38, 3098; J. Perdew, Phys. Rev. B, 1986, 33, 8822) und der def-SV(P)-Basissatz (A. Schäfer, H. Horn and R. Ahlrichs, J. Chem. Phys., 1992, 97, 2571).

Des Weiteren wird auch ein Verfahren zur Herstellung eines zuvor beschriebenen Gemisches beansprucht.

Verfahren zur Herstellung eines Gemisches, wie es zuvor beschrieben wurde. umfassend die Verfahrensschritte:
a) oxidative Kopplung gemäß Reaktionsschema A: 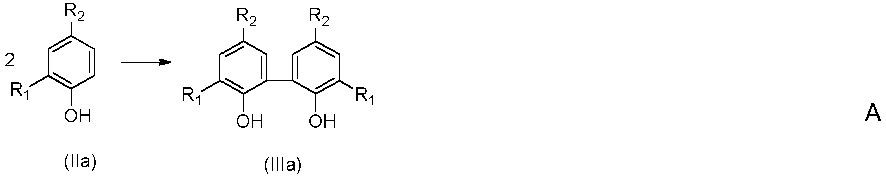
b) oxidative Kopplung gemäß Reaktionsschema B: 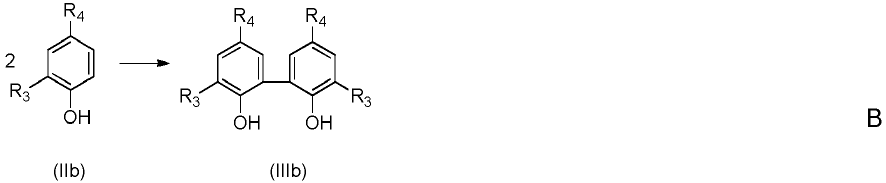
c) Umsetzung des Produktes aus a) mit PCl₃ gemäß Reaktionsschema C: 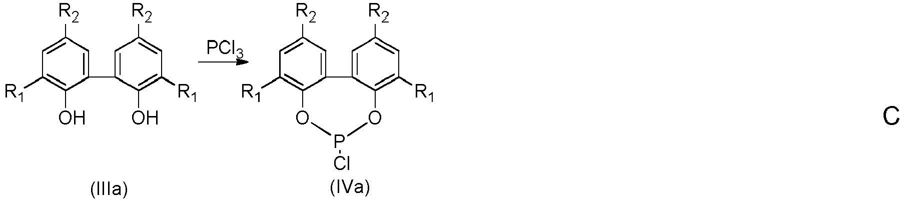
d) Umsetzung des Produktes aus b) mit dem Produkt aus c) zu einem Bisphosphit.
e) Wiederholung der Verfahrensschritte a) bis d), wobei die Reste R1 bis R4 so gewählt sind, dass sie nicht alle identisch zum ersten Durchgang sind,
f) Mischen der Verbindungen aus dem ersten und dem zweiten Durchgang.

In einer Variante des Verfahrens umfasst dieses zusätzlich den Verfahrensschritt g) Umsetzung mit M zu (Ic') und (Ic"), wobei M ausgewählt ist aus Fe, Ru, Os, Co, Rh, Ir, Ni,
Pd, Pt.

### Beispiele

### Allgemeine Reaktionsgleichung zur Synthese von Verbindung (1Ia')

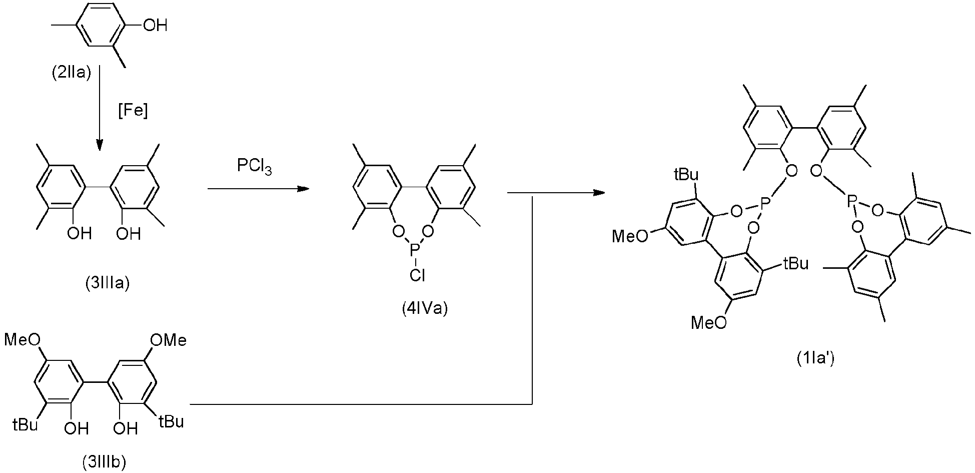

### Abkürzungen:

VE-Wasser = demineralisiertes Wasser
KPG = Kerngezogenes Präzisions-Glasgerät
ACN = Acetonitril
EtOAc = Ethylacetat
acac = acetylacetonat
NEt₃ = Triethylamin
TIPB = 1,2,4,5-Tetraisopropylbenzol

### Synthese des 2,2'-Bis(3,5-dimethylphenol) (3IIIa)

Das als Vorstufe eingesetzte Biphenol (3IIIa) wurde nach folgender Synthesevorschrift hergestellt.

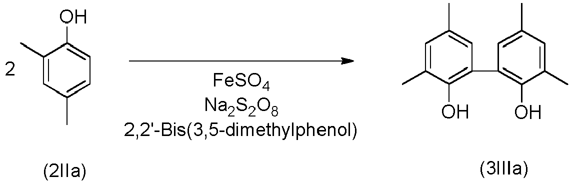

In einem 500 ml Schlenk mit KPG-Rührer, Zwischenaufsatz und Glasrührer wurden 1,42 g (0,005 mol) Eisen(II)-sulfatheptahydrat und 12,35 g (0,1 mol) 2,4-Dimethylphenol in 150 ml VE-Wasser und 5 ml Cyclohexan vorgelegt und auf 40 °C erwärmt.
In einem 100 ml Becherglas löste man 25.36 g (0,146 mol) Natriumperoxodisulfat in 80 ml VE-Wasser. Zum Start der Reaktion wurde eine kleine Portion Na₂S₂O₈-Lösung zum Phenol gegeben. Anschließend wurde alle 10 min eine kleinere Portion der Lösung hinzugegeben. Nach 30 min war die Na₂S₂O₈-Lösung Zugabe beendet.
Nach einer Reaktionszeit von 5h wurde zur Reaktionslösung 300 ml Cyclohexan und 200 ml Wasser hinzugegeben, 20 min rühren gelassen, dann warm in den Scheidetrichter überführt. Die organische Phase wurde abgetrennt und bis zur Trockene eingeengt. Das Produkt (3IIIa) konnte in 69%iger Ausbeute (10,6 g) erhalten werden.

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).
Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Mittels der ³¹P-NMR wurde der Gehalt der Liganden bestimmt.

### Synthese des 2,2'-Bis-(3,5-dimethylphenol)chlorophosphits (4IVa)

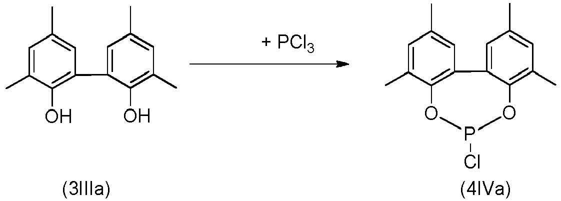

In einem sekurierten 2 L Schlenk mit Magnetrührer wurden 440 ml Phosphortrichlorid vorgelegt. In einem zweiten sekurierten 1 L Schlenk wurden 120 g 2,2'-Bis-(3,5-dimethylphenol) eingewogen und unter Rühren 500 ml getrocknetes Toluol hinzugefügt. Die Biphenol-Toluol-Suspension wurde innerhalb von 4 h bei 63°C zum Phosphortrichlorid dosiert. Nach vollständiger Zugabe wurde die Reaktionsmischung über Nacht bei Temperatur gerührt. Am nächsten Morgen wurde die Lösung in der Wärme (45°C) eingeengt und das Produkt konnten in 96,5%iger Ausbeute (153 g) erhalten werden. ³¹P-NMR: 175,59 (94,8% 2,2'-Bis-(3,5-dimethylphenol)chlorophosphit), 4,4% diverse PCI-Verbindungen, 0,8% P-H-Verbindung.

### Erfindungsgemäße Synthesevariationen zur Herstellung des reinen Liganden (1Ia')

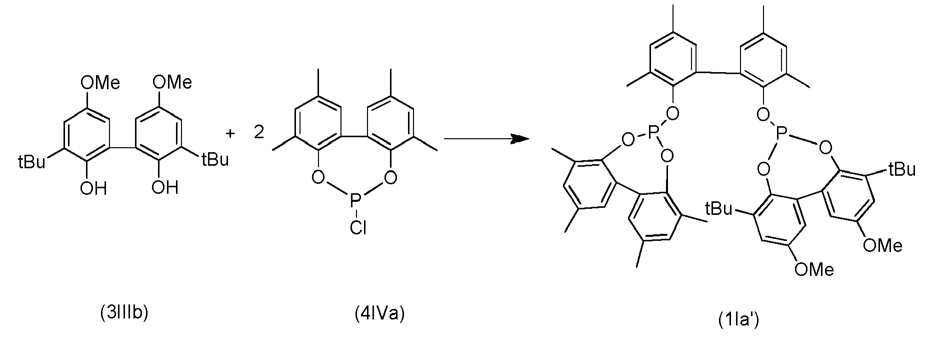

### Variante 1: ACN/NEt₃

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem Triethylamin (0,29 mol) versetzt. Dann wurde langsam die Biphenol/Triethylamin-Lösung zu der Chlorophosphitlösung getropft. Nach einer Nachreaktionszeit von 1h wurde die Reaktionslösung über Nacht bei 45°C gerührt.
Dieser Feststoff wurde in entgastem ACN 1.5h bei 75°C gerührt und gefrittet und mit warmem ACN nachgewaschen. Anschließend wurde das Produkt in getr. Toluol 1.5h bei 35°C gerührt und gefrittet. Das Zielprodukt konnte als weißer Feststoff (33 g, 66%) erhalten werden. ³¹P-NMR (202,4 MHz, toluene-d₈): 142,5 und 140,9 (100%)

### Variante 2: EtOAc/NEt₃

In einem 100 ml Schlenk wurde unter Schutzgas 7,3 g (21,0 mmol) 2,2'-Bis-(3,5-dimethylphenyl)chlorophosphit in 15 ml entgastem Ethylacetat gelöst und auf 35°C erwärmt. In einem zweiten Schlenk (100 ml) wurden 3,9 g (9,5 mmol) 3,3'-Di-tert.-butyl-5,5'-dimethoxy-[1,1'-biphenyl]-2,2'diol in 7,0 ml NEt₃ gelöst. Anschließend wurde die Biphenol/Triethylamin-Lösung langsam innerhalb von 20 Minuten zu der Chlorophosphitlösung getropft. Die Lösung wurde eine weitere Stunde bei 35°C und anschließend über Nacht bei 45°C gerührt.

Dieser Feststoff wurde in entgastem ACN 1.5h bei 75°C gerührt und gefrittet und mit warmen ACN nachgewaschen. Anschließend wurde das Produkt in getr. Toluol 1.5h bei 35°C gerührt und gefrittet.

Das Zielprodukt konnte als weißer Feststoff (5.0 g, 58%) erhalten werden. ³¹P-NMR (202,4 MHz, toluene-d₈): 142,5 und 140,9 (100%).

### Variante 3: EtOAc/Pyridin

In einem 250 ml Schlenk wurde unter Schutzgas 10,07 g (31,0 mmol) 2,2'-Bis-(3,5-dimethylphenyl)chlorophosphit in 20 ml entgastem Ethylacetat gelöst und auf 45°C erwärmt. In einem zweiten Schlenk (50 ml) wurden 5,54 g (15 mmol) 3,3'-Di-tert.-butyl-5,5'-dimethoxy-[1,1'-biphenyl]-2,2'diol in 26 ml Ethylacetat und 5,2 ml entgastem Pyridin gelöst. Anschließend wurde die Biphenol/Pyridin-Lösung langsam innerhalb von 30 Minuten zu der Chlorophosphitlösung getropft. Die Lösung wurde über Nacht bei 45°C gerührt.
Am nächsten Tag wurde die Lösung filtriert und der Feststoff mit ACN gewaschen. Das Zielprodukt konnte als weißer Feststoff (4,2g, 31%) erhalten werden. ³¹P-NMR (202,4 MHz, toluene-d₈): 142,2 und 141,1 (100%).

### Variante 4: Durchführung eines Tieftemperaturversuches bei -20°C

In einem 250 ml Schlenk wurde unter Schutzgas 8,0 g (0,025 mol) 2,2'-Bis-(3,5-dimethylphenyl)chlorophosphit in 30 ml entgastem ACN gelöst und auf -20°C gekühlt. In einem zweiten Schlenk (100 ml) wurden 4,32 g (0,012 mol) 3,3'-Di-tert.-butyl-5,5'-dimethoxy-[1,1'-biphenyl]-2,2'diol in 30 ml entgastem ACN gelöst und unter Rühren mit 8,5 ml entgastem Triethylamin versetzt. Dann wurde langsam die Biphenol-Triethylamin-Lösung bei -20°C zu der Chlorophosphitlösung getropft. Nach vollständiger Zugabe wurde für weitere 4 Stunden bei -20 °C weiter gerührt. Über Nacht wurde die Reaktionslösung bis zum Morgen bei -10 °C gerührt. Dieses Vorgehen, Reaktionstemperatur über Tag bei -20 °C und über Nacht bei -10 °C, wurde 3 Tage wiederholt durchgeführt. Im Anschluss daran wurde der Reaktionsansatz innerhalb von 3 Stunden auf RT gebracht.
Anschließend wurde die Lösung filtriert und der Feststoff mit kaltem ACN gewaschen. Das Zielprodukt konnte als weißer Feststoff (7,6 g, 70%) erhalten werden. ³¹P-NMR (202,4 MHz, toluene-d₈): 142,5 und 140,9 (100%).

Das unsymmetrische Bisphosphit (1Ia') konnte somit völlig überraschend und entgegen dem Stand der Technik auch bei tiefen Temperaturen in guten Ausbeuten und exzellenter Reinheit erhalten werden.

### Aufreinigung des Liganden (1Ia'):

Neben dem Suspendieren des Liganden in verschiedenen Lösungsmitteln (siehe obiges Beispiel) ist es auch möglich, den Liganden mittels Umkristallisation zu reinigen. Diese Umkristallisation erfolgte nach WO 2012095255. Anstelle o-Xylol kann auch Toluol zur Umkristallisation in analoger Weise verwendet werden.

### Erfindungsgemäße Synthese des Liganden (1Ia") - Allgemeine Reaktionsgleichung

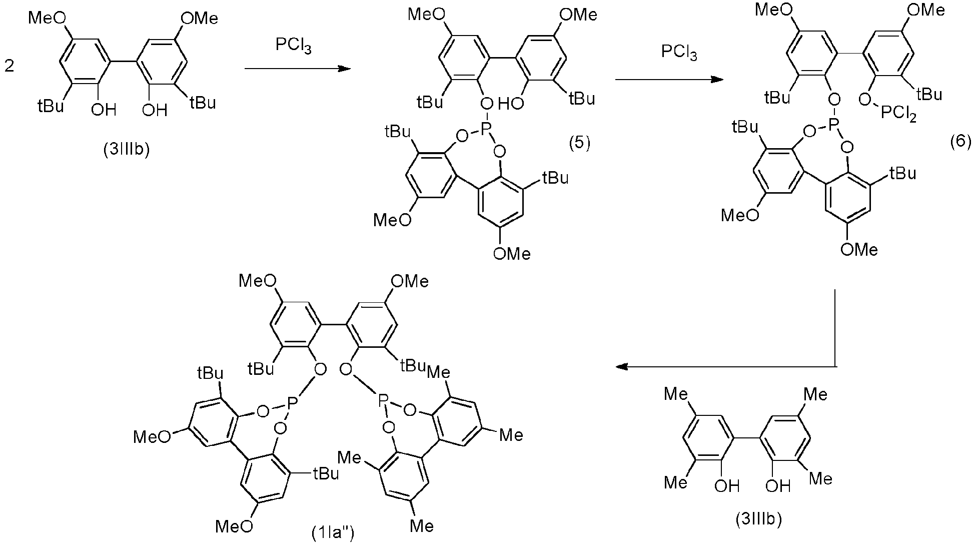

### Synthese des Phosphits (5)

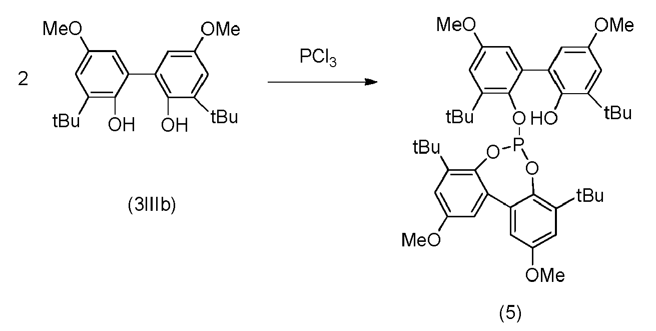

In einem sekurierten 1000 ml Schlenk wird 400 ml getrocknetes Toluol vorgelegt und mittels Spritze 8.9 ml (0.1 mol) Phosphortrichlorid hinzugegeben und auf 0 °C abgekühlt.

In einem 500 ml Schlenk werden 71.6 g (0.2 mol) 3,3'-Ditert.-butyl-2,2'-dihydroxy-5,5'-dimethoxybiphenyl abgewogen und in 325 ml getrocknetem Toluol und 49 ml (0.35 mol) getrocknetem Triethylamin gelöst.

Nun wird die Biphenol/Et₃N/Toluol-Suspension innerhalb von 2.5 h zur 0 °C gekühlten PCl₃/Toluol-Lsg getropft und über Nacht bei RT reagieren gelassen.

Am nächsten Morgen wurde der entstandene Feststoff filtriert, mehrmals mit getrocknetem Toluol nachgewaschen und das Filtrat bis zur Trockene eingeengt. Um einen weißen Feststoff zu erhalten, wurden weitere Male mit ACN nachgewaschen. Das Zielprodukt konnte so in 79.5%iger Ausbeute (59,1 g) erhalten werden.

### Synthese des Diorganophosphitdichlorophosphits (6)

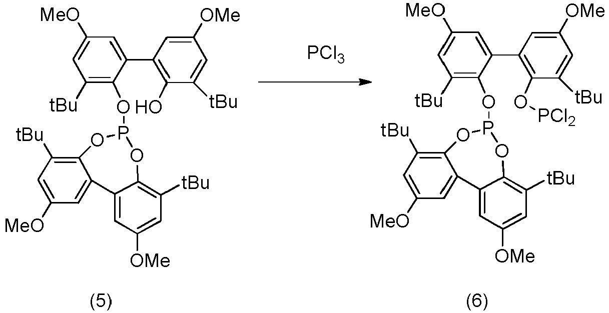

In einem sekurierten 250 ml Schlenk wurden 42 g (0,056 mol) des Phosphits (5) abgewogen und unter Rühren 275 ml getrocknetes Toluol und 17 ml (0,168 mol) getrocknetes Triethylamin hinzugegeben.

In einem zweiten 1000 ml Schlenk wurden zunächst 200 ml getrocknetes Toluol vorgelegt und anschließend 14,76 ml (0,169 mol) Phosphortrichlorid zugegeben. Anschließend wurde unter kräftigem Rühren tropfenweise zu der Phosphortrichlorid/Toluol-Lösung die zuvor hergestellte Phosphit/Amin/Toluol-Lösung innerhalb von 30 Minuten bei RT getropft. Nach vollständiger Zugabe wurde die Reaktionsmischung für 6 h auf 80 °C erwärmt und über Nacht auf RT kommen lassen.

Am nächsten Morgen wurde filtriert, mit 50 ml getrocknetem Toluol nachgewaschen und das Filtrat bis zur Trockne eingeengt. Das Produkt konnte in 89%iger Ausbeute (45,6 g) erhalten werden.

### Erfindungsgemäße Synthese des Liganden (1Ia")

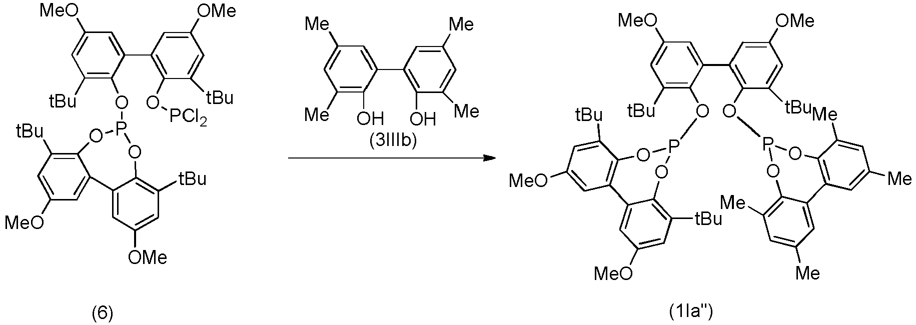

In der Glove-Box wurde in einem sekurierten 100 ml Schlenk 3,08 g (0,0036 mol) Diorganophosphitdichlorophosphit (6) eingewogen und anschließend in 35 ml getrocknetem Toluol gelöst.

In einem zweiten sekurierten 250 ml Schlenk wurden 0,872 g (0,0036 mol) 2,2'-Bis(3,5-dimethylphenol) und 1,09 g (0,01 mol) getrocknetes Triethylamin in 35 ml Toluol gelöst.

Dann wurde zur Biphenyl-Triethylamin-Lösung langsam und stetig unter kräftigem Rühren bei RT das Diorganophosphitdichlorophosphit (6) hinzugetropft. Anschließend wurde die Reaktionsmischung über Nacht gerührt.

Zur Aufarbeitung wurde der entstandene Feststoff am nächsten Morgen filtriert und zweimal mit 5 ml getrocknetem Toluol nachgewaschen. Das erhaltene Filtrat wurde dann bis zur Trockne eingeengt. Das Zielprodukt konnte als weißer Feststoff erhalten (2,59 g; 71%) erhalten werden.

### Arbeitsvorschrift für die Hydroformylierungsexperimente

### Versuchsbeschreibung - allgemein

Die Versuche wurden in 100 ml-Autoklaven der Fa. Parr Instrument durchgeführt. Die Autoklaven sind mit einer elektrischen Beheizung ausgerüstet. Die Druckkonstanthaltung erfolgt über Massedurchflußmesser und Druckregler. Während der Versuchszeit kann über eine Spritzenpumpe eine genau definierte Eduktmenge unter Reaktionsbedingungen eingespritzt werden. Über Kapillarleitungen und HPLC-Ventile können während der Versuchszeit Proben gezogen und sowohl über GC- als auch über LC-MS-Analytik untersucht werden.

### Erfindungsgemäße Ergebnisse der Testung der verschiedenen Ligandenmischungen der Liganden (1Ia') und (1Ia") in der Hydroformylierung^{[a]}:

Das Mischen der beiden Bisphosphite (1Ia') und (1Ia") erfolgte manuell vor dem Einsatz in der Hydroformylierungsreaktion.

**Tabelle 4:**

| **Nr** | **Liganden** | **Gehalt an Liganden** | **Verhältnis der Liganden in [%]^{[e]}** | **Pentanalselektivität in mol [%]^{[b]}** | **Ausbeute In [%]^{[b]}** |
|---|---|---|---|---|---|
| 1 | Ligand (1Ia') | 100% | L1Ia': 100% | 94,0^{[c]} | 92,9^{[c]} |
| 2 | Ligand (1Ia") | 100% | L1Ia": 100% | 53,2 | 76,2 |
| 3* | Ligand (1Ia') + Ligand (1Ia") | L1Ia':L1Ia":Rh 2,3:2,2:1 | L1Ia': 51% + L1Ia": 49% | 79,6 | 93,8 |
| 4* | Ligand (1Ia') + Ligand (1Ia") | L1Ia':L1a":Rh 3,3:1,3:1 | L1Ia': 72% + L1Ia": 28% | 80,5 | 93,8 |
| 5* | Ligand (1Ia') + Ligand (1Ia") | L1Ia:L1Ia":Rh 1,2:2,8:1 | L1Ia': 30% + L1Ia": 70% | 79,6 | 91,0 |

| | | | | | |
|---|---|---|---|---|---|
| * erfindungsgemäß | | | | | |

[a] Bedingungen: cis-2-Buten, Rh(acac)(CO)₂, Toluol, Verbindung (7), 120°C, 20 bar CO/H₂ (1:1), 1,2,4,5-Tetra-isopropyl-benzol oder Mesitylen als interner GC-Standard. [b] GC-Analyse mit 1,2,4,5-Tetra-isopropyl-benzol oder Mesitylen als interner GC-Standard. [c] Pentanalselektivität und Ausbeute in [%]. [d] Aldehydausbeute in [%]. [e] Verhältnis der beiden Liganden zueinander in Molprozent und auf 100% normiert.

Bei einem Vergleich der verschiedenen Ligandenmischungen aus den unsymmetrischen Liganden (1Ia') und (1Ia") (Tabelle 4, Einträge 4-6) mit dem Hydroformylierungsergebnis des reinen Liganden (1Ia") (Tabelle 4, Eintrag 2) zeigt sich, dass die Mischungen gute Pentanalselektivitäten und Ausbeuten aufweisen, die deutlich höher sind als von dem reinen Liganden (1Ia") (Tabelle 4, Einträge 2 und 3-5).

Diese guten Ausbeuten und Selektivitäten von unsymmetrischen Bisphosphiten sind völlig überraschend und entgegen dem Stand der Technik, in dem unsymmetrisch aufgebaute Bisphosphite bei der Verwendung als Ligand in der übergangsmetallkatalysierten Hydroformylierung deutlich geringere Reaktivitäten und geringere n-Regioselektivität aufweisen (siehe in Rhodium-catalyzed Hydroformylation, ed. by P.W.N.M. van Leeuwen et C. Claver, KluwerAcademic Publishers 2006, AA Dordrecht, NL, Seite 45-46). Weiterhin konnte durch die mit einem weiteren unsymmetrischen Bisphosphit eine deutlich höhere Pentanalselektivität erzeugt werden als von dem reinen Liganden (1Ia").

Es konnte somit gezeigt werden, dass in Hydroformylierungsreaktionen auch Ligandenmischungen aus unsymmetrischen Bisphosphiten eingesetzt werden können, die die technische Aufgabe erfüllen.

Für die nachfolgenden Versuche wurden der Ligand (1Ia") sowie Kombinationen aus den beiden unsymmetrischen Liganden (1Ia') und (1Ia") untersucht.

### Beispiel 15 (nicht erfindungsgemäss)

In einem 100 ml-Autoklaven der Fa. Parr Instruments wurde bei 120 °C und 20 bar 6,0 g cis-2-Buten hydroformyliert. Als Precursor wurden 0.0049 g Rh(acac)(CO)₂ in 44.38 g Toluol vorgelegt. Als Ligand wurden 0.0783 g Ligand (1Ia") in der Katalysatoransatzlösung eingesetzt. Als organisches Amin wurden 0.0392 g der Verbindung (7) und 0.4981 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 53.2 mol-% Pentanal, 16.6 mol-% 2-Methylbutanal und 3.19 mol-% n-Butan gebildet. Die Regioselektivität zu n-Pentanal beträgt 76.2 %.

### Beispiel 16

In einem 100 ml-Autoklaven der Fa. Parr Instruments wurde bei 120 °C und 20 bar 5.9 g cis-2-Buten hydroformyliert. Als Precursor wurden 0.0045 g Rh(acac)(CO)₂ in 43.5 g Toluol vorgelegt. Als Ligand wurden 0.036 g Ligand (1Ia') und 0.0383 g Ligand (1Ia") (molares Verhältnis L1Ia' : L1a" : Rh = 2.3 : 2.2 : 1) in der Katalysatoransatzlösung eingesetzt. Als organisches Amin wurden 0.0374 g der Verbindung (7) und 0.5096 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 79.6 mol-% Pentanal, 5.27 mol-% 2-Methylbutanal und 3.65 mol-% n-Butan gebildet. Die Regioselektivität zu n-Pentanal beträgt 93.8 %.

### Beispiel 17

In einem 100 ml-Autoklaven der Fa. Parr Instruments wurde bei 120 °C und 20 bar 6.3 g cis-2-Buten hydroformyliert. Als Precursor wurden 0.0049 g Rh(acac)(CO)₂ in 45.0 g Toluol vorgelegt. Als Ligand wurden 0.0568 g Ligand (1Ia') und 0.0249 g Ligand (1Ia") (molares Verhältnis L1Ia' : L1a" : Rh = 3.3 : 1.3 : 1) in der Katalysatoransatzlösung eingesetzt. Als organisches Amin wurden 0.0376 g der Verbindung (7) und 0.5103 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 80.5 mol-% Pentanal, 5.29 mol-% 2-Methylbutanal und 3.08 mol-% n-Butan gebildet. Die Regioselektivität zu n-Pentanal beträgt 93.8 %.

### Beispiel 18

In einem 100 ml-Autoklaven der Fa. Parr Instruments wurde bei 120 °C und 20 bar 5.6 g cis-2-Buten hydroformyliert. Als Precursor wurden 0.0054 g Rh(acac)(CO)₂ in 45.6 g Toluol vorgelegt. Als Ligand wurden 0.0215 g Ligand (1Ia') und 0.0587 g Ligand (1Ia") (molares Verhältnis L1Ia' : L1a" : Rh = 1.2 : 2.8 : 1) in der Katalysatoransatzlösung eingesetzt. Als organisches Amin wurden 0.0364 g der Verbindung (7) und 0.5073 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 12 Stunden gezogen. Es wurden 79.6 mol-% Pentanal, 7.9 mol-% 2-Methylbutanal und 3.63 mol-% n-Butan gebildet. Die Regioselektivität zu n-Pentanal beträgt 91.0 %.

## Patentansprüche

1. Gemisch umfassend die unsymmetrischen Verbindungen (la') und (la"): 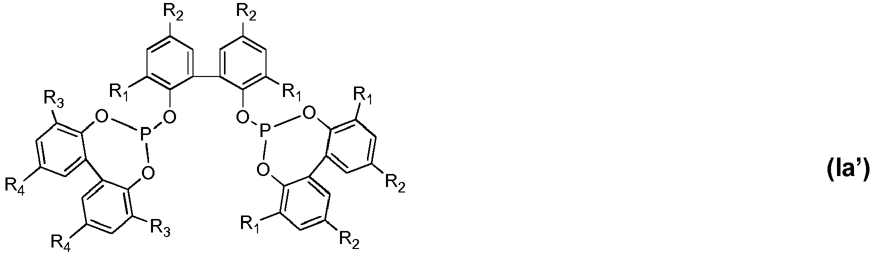 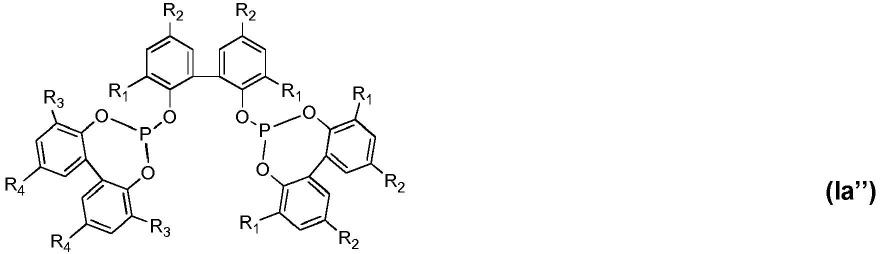 wobei
R1 ausgewählt ist aus -Me, -tBu, -OMe;
R2 ausgewählt ist aus -Me, -tBu, -OMe;
R3 ausgewählt ist aus -Me, -tBu, -OMe;
R4 ausgewählt ist aus -Me, -tBu, -OMe;
und P weitere Bindungen eingehen kann,
und die Verbindungen (la') und (la") nicht identisch sind.

2. Gemisch nach Anspruch 1
wobei der Gehalt an Verbindung (la') in einem Bereich von 99,5 bis 0,5 Mol-%, der Gehalt an Verbindung (la") in einem Bereich von 0,5 bis 99,5 Mol-% liegt.

3. Gemisch nach einem der Ansprüche 1 oder 2,
umfassend die Bisphosphite der Formeln (Ib') und (Ib"): 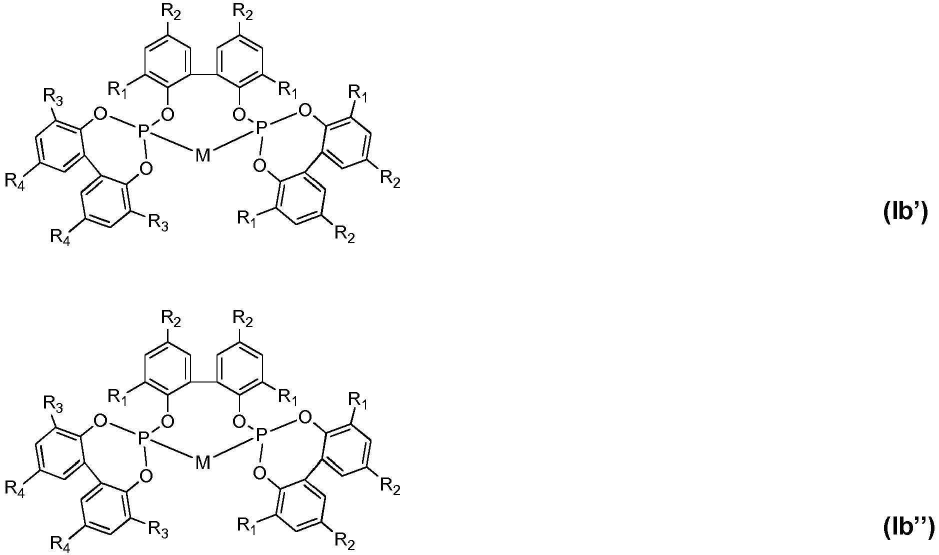 wobei M ausgewählt ist aus Fe, Ru, Os, Co, Rh, Ir, Ni, Pd, Pt, und M zusätzliche Bindungen eingehen kann,
und die Verbindungen (Ib') und (Ib") nicht identisch sind.

4. Gemisch nach einem der Ansprüche 1 bis 3,
umfassend die Verbindungen der Formeln (Ic') und (Ic"): 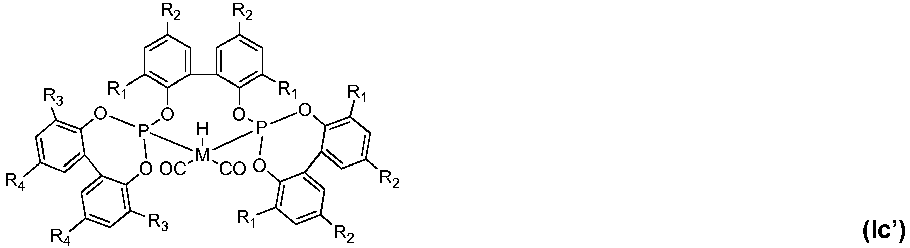 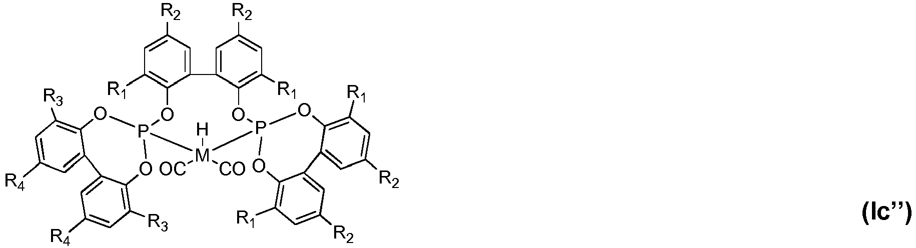 wobei M ausgewählt ist aus Fe, Ru, Os, Co, Rh, Ir, Ni, Pd, Pt, und und die Verbindungen (Ic') und (Ic") nicht identisch sind.

5. Gemisch nach Anspruch 4,
welches zusätzlich mindestens eine Verbindung (la') oder (la") umfasst, die nicht an M gebunden sind.

6. Gemisch nach einem der Ansprüche 3 bis 5,
wobei M für Rh steht.

7. Gemisch nach einem der Ansprüche 1 bis 6,
wobei R1 -Me ist, und R3 nicht -Me ist.

8. Gemisch nach einem der Ansprüche 1 bis 7,
wobei R2 -Me ist, und R4 nicht -Me ist.

9. Gemisch nach einem der Ansprüche 1 bis 8,
wobei R1 und R2 -Me sind.

10. Gemisch nach einem der Ansprüche 1 bis 6,
wobei R1 -tBu ist, und R3 nicht -tBu ist.

11. Gemisch nach einem der Ansprüche 1 bis 6,
wobei R2 -OMe ist, und R4 nicht -OMe ist.

12. Zusammensetzung umfassend:
- ein Gemisch nach einem der Ansprüche 1 bis 11,
- eine weitere Komponente ausgewählt aus: Basen, organische Amine, Epoxide, Pufferlösungen, Ionenaustauscher.

13. Zusammensetzung nach Anspruch 12,
wobei das organische Amin zumindest eine 2,2,6,6-Tetramethylpiperidineinheit aufweist.

14. Verfahren zur Herstellung eines Gemisches nach Anspruch 1,
umfassend die Verfahrensschritte:
a) oxidative Kopplung gemäß Reaktionsschema A: 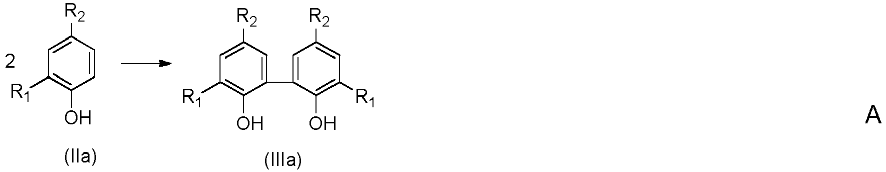
b) oxidative Kopplung gemäß Reaktionsschema B: 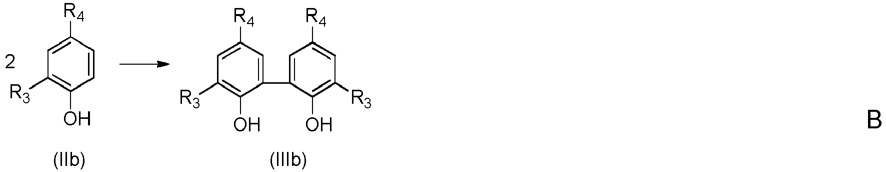
c) Umsetzung des Produktes aus a) mit PCl₃ gemäß Reaktionsschema C: 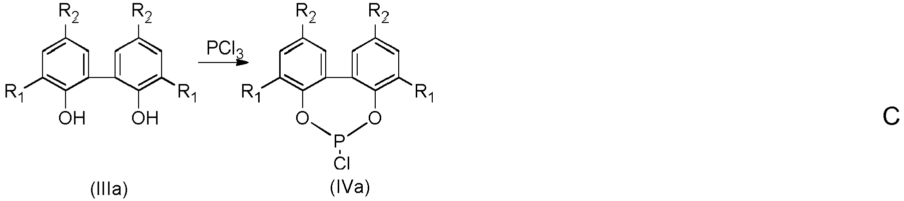
d) Umsetzung des Produktes aus b) mit dem Produkt aus c) zu einem Bisphosphit.
e) Wiederholung der Verfahrensschritte a) bis d), wobei die Reste R1 bis R4 so gewählt sind, dass sie nicht alle identisch zum ersten Durchgang sind,
f) Mischen der Verbindungen aus dem ersten und dem zweiten Durchgang.

15. Verfahren nach Anspruch 14,
zusätzlich umfassend den Verfahrenschritt:
g) Umsetzung mit M zu (Ic') und (Ic"), wobei M ausgewählt ist aus Fe, Ru, Os, Co, Rh, Ir, Ni, Pd, Pt.

16. Verwendung eines Gemisches nach einem der Ansprüche 1 bis 11 als Katalysator in einer Hydroformylierungsreaktion von ungesättigten Verbindungen und deren Gemischen.

17. Verfahren zur Hydroformylierung von ungesättigten Verbindungen und deren Gemischen unter Verwendung:
- einer Zusammensetzung nach einem der Ansprüche 12 oder 13, und
- eines Gasgemisches umfassend Kohlenmonoxid und Wasserstoff.

18. Verfahren nach Anspruch 17, wobei die ungesättigten Verbindungen und deren Gemische ausgewählt sind aus:
- Kohlenwasserstoffgemischen aus Dampfspaltanlagen;
- Kohlenwasserstoffgemischen aus katalytisch betriebenen Spaltanlagen;
- Kohlenwasserstoffgemischen aus Oligomerisierungsprozessen;
- Kohlenwasserstoffgemischen umfassend mehrfach ungesättigte Verbindungen;
- ungesättigten Carbonsäurederivaten.

19. Verfahren nach Anspruch 18,
**dadurch gekennzeichnet, dass** die Kohlenwasserstoffgemische ungesättigte Verbindungen mit 2 bis 30 Kohlenstoffatomen aufweisen.

## Claims

1. Mixture comprising the unsymmetric compounds (Ia') and (Ia'') : 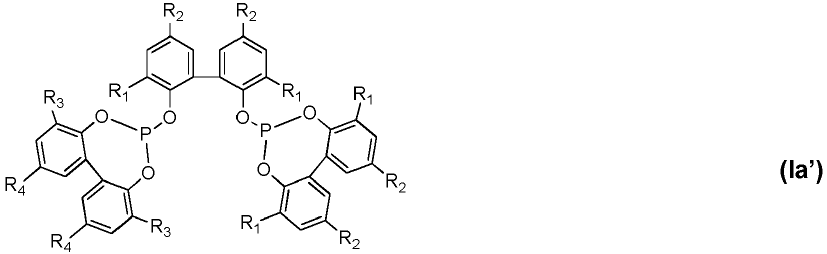 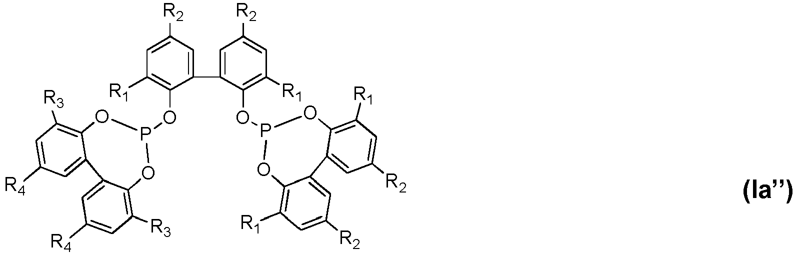 where
R1 is selected from -Me, -tBu, -OMe;
R2 is selected from -Me, -tBu, -OMe;
R3 is selected from -Me, -tBu, -OMe;
R4 is selected from -Me, -tBu, -OMe;
and P can enter into further bonds,
and the compounds (Ia') and (Ia'') are not identical.

2. Mixture according to Claim 1,
wherein the content of compound (Ia') is within a range from 99.5 to 0.5 mol%, and the content of compound (Ia'') within a range from 0.5 to 99.5 mol%.

3. Mixture according to either of Claims 1 and 2, comprising the bisphosphites of the formulae (Ib') and (Ib'') : 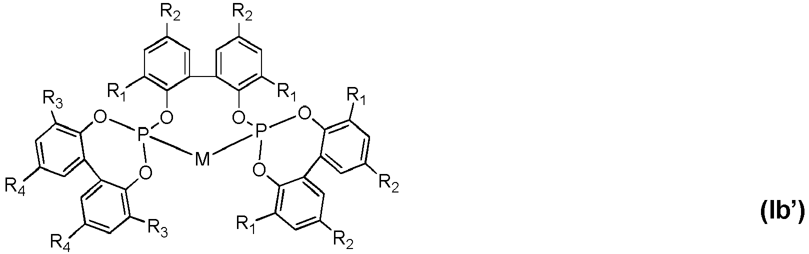 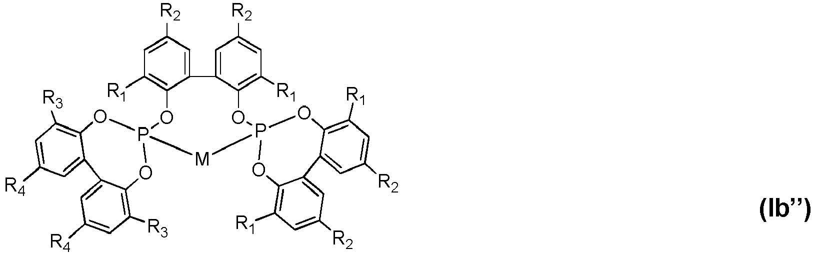
where M is selected from Fe, Ru, Os, Co, Rh, Ir, Ni, Pd, Pt,
and M can enter into additional bonds,
and the compounds (Ib') and (Ib'') are not identical.

4. Mixture according to any of Claims 1 to 3, comprising the compounds of the formulae (Ic') and (Ic'') : 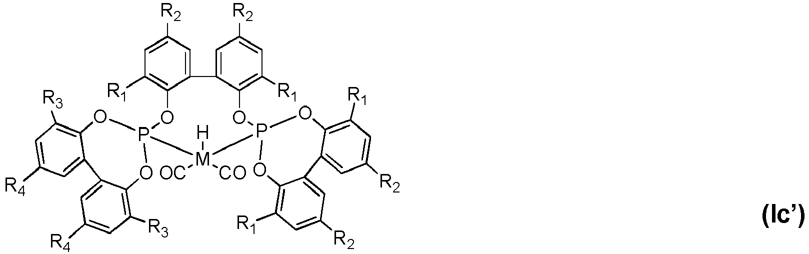 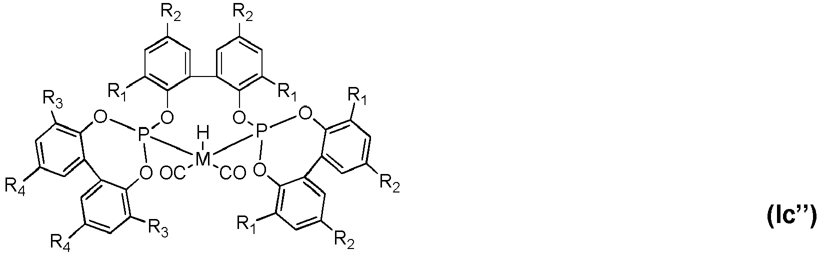 where M is selected from Fe, Ru, Os, Co, Rh, Ir, Ni, Pd, Pt,
and the compounds (Ic') and (Ic'') are not identical.

5. Mixture according to Claim 4,
which additionally comprises at least one compound (Ia') or (Ia") not bonded to M.

6. Mixture according to any of Claims 3 to 5,
where M is Rh.

7. Mixture according to any of Claims 1 to 6,
where R1 is -Me, and R3 is not -Me.

8. Mixture according to any of Claims 1 to 7,
where R2 is -Me, and R4 is not -Me.

9. Mixture according to any of Claims 1 to 8,
where R1 and R2 are each -Me.

10. Mixture according to any of Claims 1 to 6,
where R1 is -tBu, and R3 is not -tBu.

11. Mixture according to any of Claims 1 to 6,
where R2 is -OMe, and R4 is not -OMe.

12. Composition comprising:
- a mixture according to any of Claims 1 to 11,
- a further component selected from: bases, organic amines, epoxides, buffer solutions, ion exchangers.

13. Composition according to Claim 12,
wherein the organic amine has at least one 2,2,6,6-tetramethylpiperidine unit.

14. Process for preparing a mixture according to Claim 1,
comprising the process steps of:
a) oxidative coupling according to reaction scheme A: 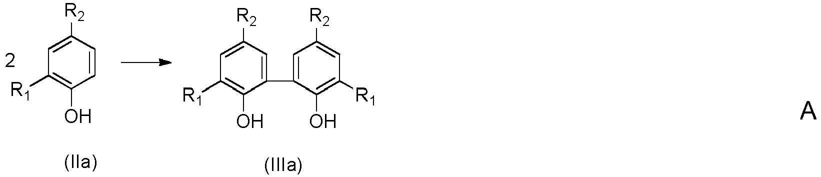
b) oxidative coupling according to reaction scheme B: 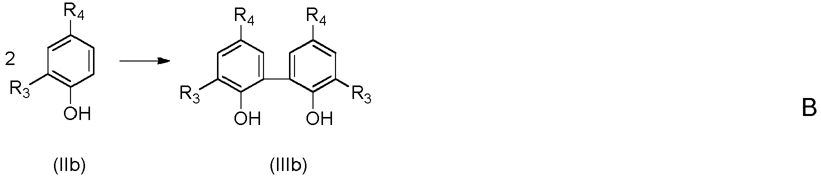
c) reaction of the product from a) with PCl₃ according to reaction scheme C: 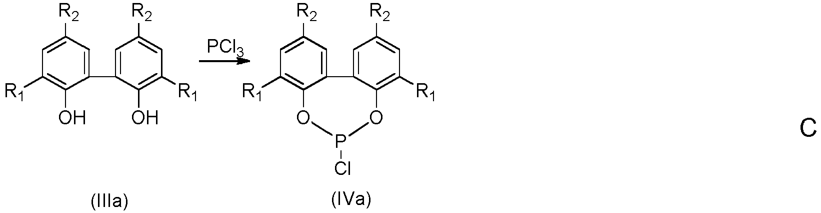
d) reaction of the product from b) with the product from c) to give a bisphosphite,
e) repetition of process steps a) to d), where the R1 to R4 radicals are selected such that they are not all identical to the first run,
f) mixing of the compounds from the first and second runs.

15. Process according to Claim 14,
additionally comprising the process step of:
g) reaction with M to give (Ic') and (Ic''), where M is selected from Fe, Ru, Os, Co, Rh, Ir, Ni, Pd, Pt.

16. Use of a mixture according to any of Claims 1 to 11 as a catalyst in a hydroformylation reaction of unsaturated compounds and mixtures thereof.

17. Process for hydroformylating unsaturated compounds and mixtures thereof using:
- a composition according to either of Claims 12 and 13, and
- a gas mixture comprising carbon monoxide and hydrogen.

18. Process according to Claim 17, wherein the unsaturated compounds and mixtures thereof are selected from:
- hydrocarbon mixtures from steamcracking plants;
- hydrocarbon mixtures from catalytically operated cracking plants;
- hydrocarbon mixtures from oligomerization operations;
- hydrocarbon mixtures comprising polyunsaturated compounds;
- unsaturated carboxylic acid derivatives.

19. Process according to Claim 18,
**characterized in that** the hydrocarbon mixtures include unsaturated compounds having 2 to 30 carbon atoms.

## Revendications

1. Mélange comprenant les composés asymétriques (Ia') et (Ia") : 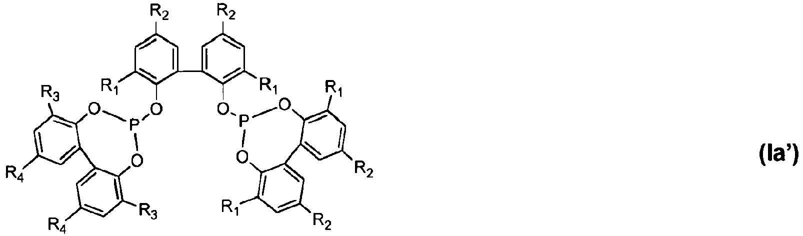 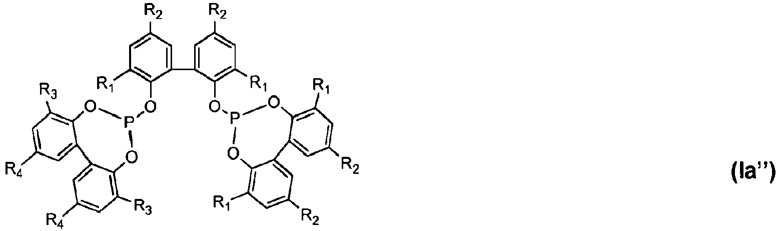 dans lesquels
R1 est choisi parmi -Me, -tBu, -OMe ;
R2 est choisi parmi -Me, -tBu, -OMe ;
R3 est choisi parmi -Me, -tBu, -OMe ;
R4 est choisi parmi -Me, -tBu, -OMe ; et
P peut former des liaisons supplémentaires
et les composés (Ia') et (Ia") ne sont pas identiques.

2. Mélanges selon la revendication 1, la teneur en composé (Ia') étant située dans une plage de 99,5 à 0,5% en mole, la teneur en composé (Ia") étant située dans une plage de 0,5 à 99,5% en mole.

3. Mélange selon l'une quelconque des revendications 1 ou 2, comprenant les bis-phosphites des formules (Ib') et (Ib") : 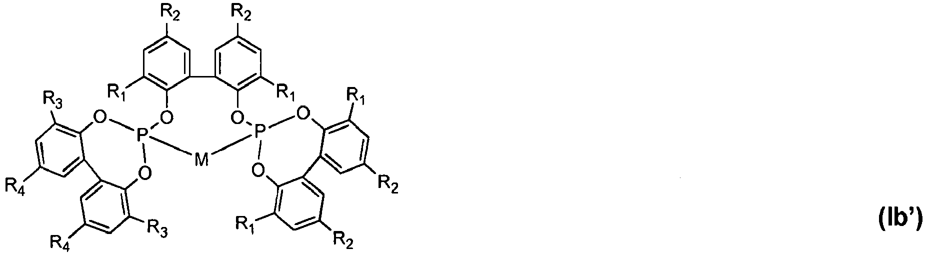 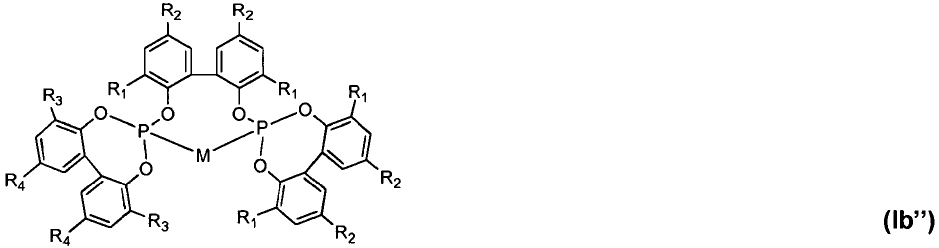 dans lesquelles M est choisi parmi Fe, Ru, Os, Co, Rh, Ir, Ni, Pd, Pt,
et M peut former des liaisons supplémentaires et les composés (Ib') et (Ib") ne sont pas identiques.

4. Mélange selon l'une quelconque des revendications 1 à 3, comprenant les composés des formules (Ic') et (Ic") : 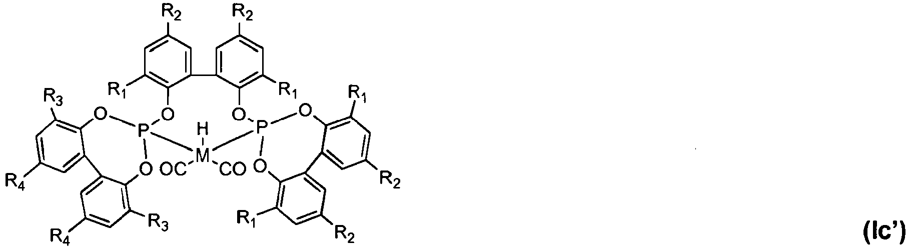 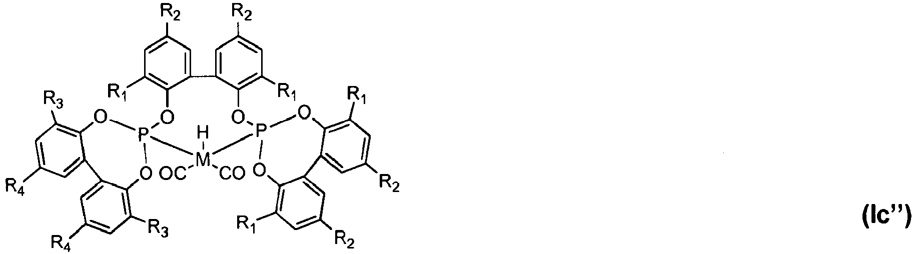 dans lesquelles M est choisi parmi Fe, Ru, Os, Co, Rh, Ir, Ni, Pd, Pt et les composés (Ic') et (Ic") ne sont pas identiques.

5. Mélange selon la revendication 4, qui comprend en outre au moins un composé (Ia') ou (Ia") qui n'est pas lié à M.

6. Mélange selon l'une quelconque des revendications 3 à 5, M représentant Rh.

7. Mélange selon l'une quelconque des revendications 1 à 6, R1 représentant -Me et R3 ne représentant pas -Me.

8. Mélange selon l'une quelconque des revendications 1 à 7, R2 représentant -Me et R4 ne représentant pas -Me.

9. Mélange selon l'une quelconque des revendications 1 à 8, R1 et R2 représentant -Me.

10. Mélange selon l'une quelconque des revendications 1 à 6, R1 représentant -tBu et R3 ne représentant pas -tBu.

11. Mélange selon l'une quelconque des revendications 1 à 6, R2 représentant -OMe et R4 ne représentant pas -OMe.

12. Composition comprenant
- un mélange selon l'une quelconque des revendications 1 à 11,
- un autre composant choisi parmi : les bases, les amines organiques, les époxydes, les solutions tampon, les échangeurs d'ions.

13. Composition selon la revendication 12, l'amine organique présentant au moins une unité 2,2,6,6-tétraméthylpipéridine.

14. Procédé pour la préparation d'un mélange selon la revendication 1, comprenant les étapes de procédé :
a) couplage par oxydation selon le schéma de réaction A : 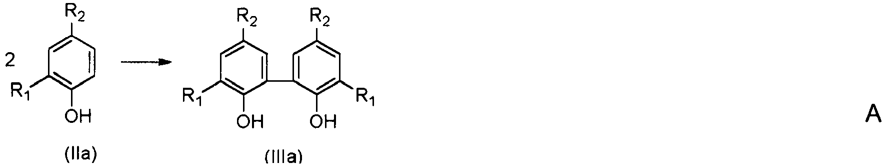
b) couplage par oxydation selon le schéma de réaction B : 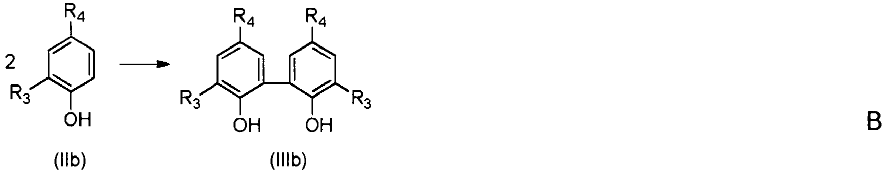
c) transformation du produit de a) avec du PCl₃ selon le schéma de réaction C : 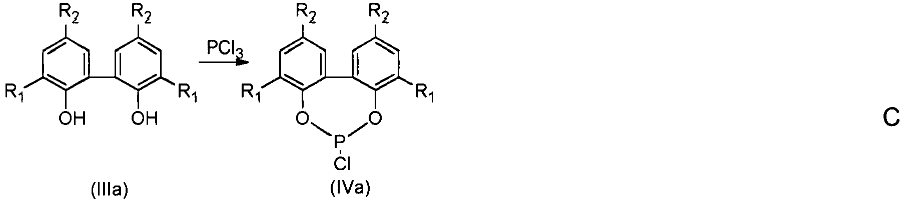
d) transformation du produit de b) avec le produit de c) en un bis-phosphite,
e) répétition des étapes de procédé a) à d), les radicaux R1 à R4 étant choisis de manière telle qu'ils ne sont pas tous identiques au premier passage,
f) mélange des composés du premier et du deuxième passage.

15. Procédé selon la revendication 14, comprenant en outre l'étape de procédé :
g) transformation avec M en (Ic') et (Ic"), M étant choisi parmi Fe, Ru, Os, Co, Rh, Ir, Ni, Pd, Pt.

16. Utilisation d'un mélange selon l'une quelconque des revendications 1 à 11 comme catalyseur dans une réaction d'hydroformylation de composés insaturés et de leurs mélanges.

17. Procédé pour l'hydroformylation de composés insaturés et de leurs mélanges avec utilisation :
- d'une composition selon l'une quelconque des revendications 12 ou 13 ;
- d'un mélange gazeux contenant du monoxyde de carbone et de l'hydrogène.

18. Procédé selon la revendication 17, les composés insaturés et leurs mélanges étant choisis parmi :
- les mélanges hydrocarbonés provenant d'installations de dissociation à la vapeur ;
- les mélanges hydrocarbonés provenant d'installations de dissociation exploitées de manière catalytique ;
- les mélanges hydrocarbonés provenant d'installations d'oligomérisation ;
- les mélanges hydrocarbonés comprenant des composés polyinsaturés ;
- les dérivés insaturés d'acide carboxylique.

19. Procédé selon la revendication 18, **caractérisé en ce que** les mélanges hydrocarbonés présentent des composés insaturés comprenant 2 à 30 atomes de carbone.
